# EUROPEAN PATENT SPECIFICATION

(11) **EP 3 903 097 B1**
(45) Date of publication and mention of the grant of the patent: **15.04.2026**
(21) Application number: 20717344.4
(22) Date of filing: 06.02.2020
(51) Int. Cl.: G01N 21/898, G01N 21/3563, G01N 21/359, G01N 33/36

(54) **PROXIMITY TRIGGERED TEXTILE SUBSTRATE CLASSIFICATION APPARATUS AND PROCEDURE**
NÄHERUNGSAUSGELÖSTE VORRICHTUNG UND ENTSPRECHENDES VERFAHREN ZUR KLASSIFIKATION VON TEXTILSUBSTRATEN
APPAREIL ET PROCÉDÉ DE CLASSIFICATION DE SUBSTRAT TEXTILE DÉCLENCHÉ PAR PROXIMITÉ

(43) Date of publication of application: 03.11.2021
(73) Proprietor: SKYLABS D.O.O., 2000 Maribor (SI)
(72) Inventor: KOTNIK, Bojan, 2000 Marbor (SI); ROTOVNIK, Tomaz, 2380 Slovenj Gradec (SI); GACNIK, Dejan, 2000 Maribor (SI); KRAMBERGER, Iztok, 2000 Maribor (SI); GOSTECNIK, Miha, 2327 Race (SI); FERLE, Aleksei Nino, 2000 Maribor (SI); MIHELIC, Ales, 1000 Ljubljana (SI); NERAL, Branko, 2212 Sentilj v Slovenskih goricah (SI); STIMULAK, Mitja, 3204 Dobrna (SI); OBU VAZNER, Kristina, 3320 Velenje (SI)
(74) Representative: Becker Kurig & Partner Patentanwälte mbB
(86) International application number: PCT/SI2020/050004
(87) International publication number: WO 2021/158181

(56) References cited:
- EP-A1- 3 569 993
- WO-A1-97/08537
- DE-A1- 19 920 592

## Description

The present invention relates to proximity triggered textile substrate classification apparatus and procedure for non-invasive textile substrate classification.

Non-invasive automated textile substrate classification is important in several domains: home appliances -including, but not limited to washing machine, washer drier, laundry ironing machine etc. - need to know the textile substrate composition of the textile products or cloths in order to properly set the parameters of the laundry process. The most important parameters of the laundry processes which depend on the textile substrate classification output are the operational temperature, bath ratio, centrifuge speed, and laundry rubbing intensity. Using inappropriate laundry process parameters for a certain textile substrate can lead to serious damage to the processed garment. Furthermore, the non-invasive textile substrate classification can also be used in the textile industry and cloth manufacture in order to verify the quality, composition, and declaration correctness of the input raw materials used during garment manufacture.

The above-used term "non-invasive" relates to the fact that the textile substrate classification of a garment needs to be performed in a way that must not destroy, damage, nor in any way degrade the state of the analysed garment. Infrared spectroscopy (especially near (NIR) and shortwave (SWIR) infrared spectroscopy) was found to be the most suitable general method for non-invasive textile substrate classification. Namely, different textile substrates were found to have rather specific IR reflectance characteristics. Therefore, the textile substrate classification can be performed by illuminating the analysed textile substrate with infrared light source and afterwards carefully analysing the IR light, which diffusely reflects from the analysed textile substrate.

There exist already several inventions which relate to some sort of non-invasive textile substrate classification procedures and their application in different home appliances.

The document WO2001046509A1 presents the inventive appliance for handling textiles, which comprises the device for identifying properties of a textile, which has been placed inside the appliance. The proposed textile identification device comprises at least one emitting unit and at least one detector unit for emitting and detecting electromagnetic radiation, especially in infrared, near-infrared, or shortwave infrared region of the electromagnetic spectrum. The textile identification result is then utilized to control the washing or drying process. This patent however does not specify precisely the procedure used for the reflectance spectrum processing and feature extraction.

The document DE102017215132A1 presents the adjustable micro-plastic filtering process for the laundry processing appliance (either washing machine or washer drier), which uses the IR spectrometer to analyse the content and properties of the micro-plastic particles inside the micro-plastic filters placed inside the exhaust water or air pipes. The patent however does not reveal the procedure used for the transmittance spectrum processing and feature extraction, nor deals with the textile substrate classification problem.

The document DE102017214852A1 presents the wireless, handheld apparatus used for garment analysis, which connects to various laundry processing home appliances (i.e. washing machine, washer drier, or ironing machine) which operational parameters can be adjusted based on the textile substrate analysis (material composition and colour). The patent describes the basic reflectance spectrometer setup of this device, which operates in the wavelength range between 700 nm and 2500 nm, preferably in between 1500 nm and 2500 nm. The colour of the textile substrate is determined by the sensor, which operates in the visible light spectral range. This patent however does not reveal the procedure used for the transmittance spectrum processing and feature extraction.

The document DE102017219806A1 presents the handheld IR-spectrometer and RGB sensor based scanner to detect and analyse the stains on garments and the procedure to use the results in a home appliance (washing machine). This patent however does not reveal the procedure used for the reflectance spectrum processing and feature extraction.

The document WO2018011176A1 presents the method and apparatus for determining especially a cleaning strategy which is based on the analysis of the reflected light ranging from ultra-violet (UV), through visual- (VIS), to near infrared (NIR) spectrum. The patent explicitly defines the textile substrate classification (colour and composition) using at least 20 spectral channels, out of which at least one channel lies outside of the visible spectrum. The patent also proposes the procedure to determine and conduct the laundry cleaning based on the optical sensor's output. The patent however does not define the feature extraction procedure neither defines how the textile substrate classification is performed.

The document WO2018086871A1 presents a spectrometer, a system containing a spectrometer and a domestic appliance, and the method for the operation thereof. According to the patent, the spectrometer is identified by embedding at least one light source, light detector, autonomous supply and the module for wireless data transmission in a housing which can various shapes, including ball, ellipsoid etc. The purpose of the spectrometer is not limited and can be used in various home appliances (washing machine, washer drier, dishwasher, etc.). The spectrometer can also operate in reflective or transmissive mode.

The document EP3569993A1 presents a handheld scanning device for determining spectral data regarding an item. The device comprises a user interface, a spectral sensor, a proximity sensor and a control unit configured to provide control over the automatic, distance triggered scanning process. The purpose of the spectrometer is not limited and can be used in various home appliances (washing machine, washer drier, dishwasher etc.). The automated scanning process employs adaptive distance thresholding and is further configured based on previous measurements, which provides flexibility when scanning several different items.

The document WO1997008537A1 presents a handheld device for light reflectance measurement of samples. The device comprises a housing including a window, a reflectance detector, an acousto-optic tuneable filter, a light source, means for generating a light beam, alignment means for optical alignment and an electric circuit for accepting a signal from the detector. The purpose of this configuration is to provide suitable spectral measurement means with the intent of non-invasive sample classification.

The document DE19920592A1 presents a method for automatic classification of textile substrate samples. The method utilises a standard reflective based NIR spectrometer setup. The method conducts several consecutive spectral measurements and classifications in order to form a final, time-averaged classification result.

The present invention relates to proximity triggered textile substrate classification apparatus and procedure for non-invasive textile substrate classification. The invention is defined by independent claims 1 and 11. Further embodiments are defined by the dependent claims. The following disclosure serves a better understanding of the present invention.

The invention is described in more detail on the basis of design examples and the corresponding figures, which show:

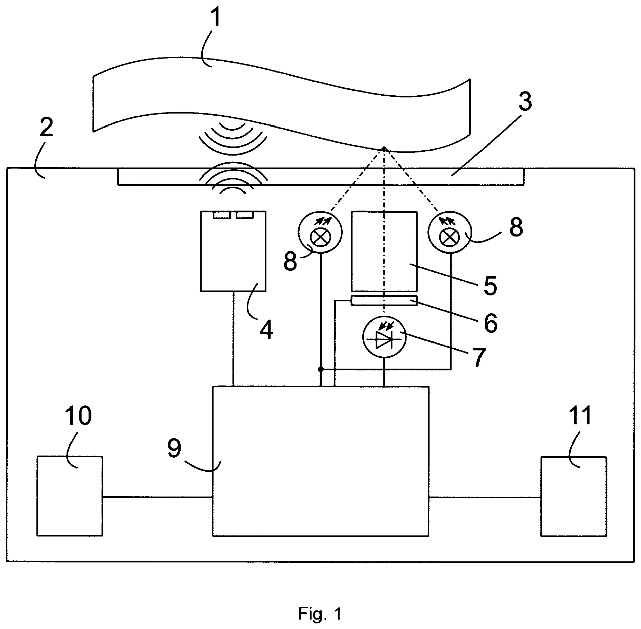

- Fig. 1: block diagram of the proximity triggered textile substrate classification apparatus according to invention;

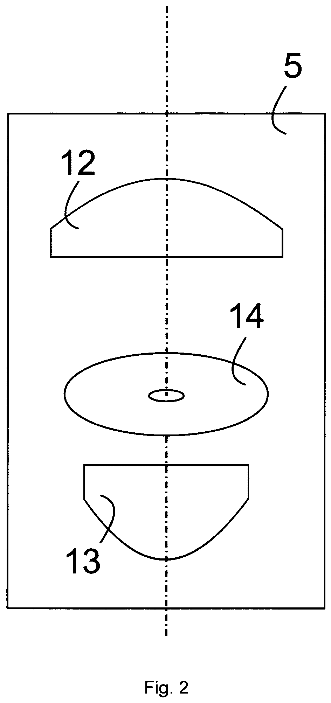

- Fig. 2: schematic representations of the optical module 5;

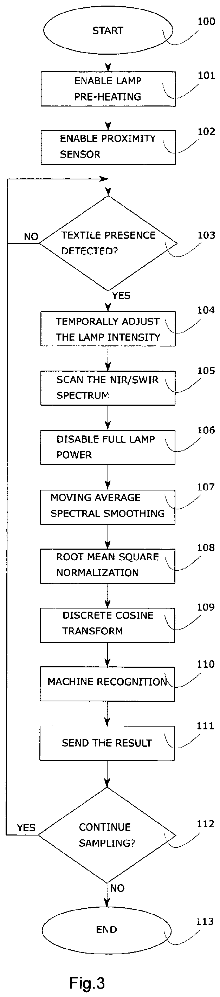

- Fig. 3: flow chart of the procedure for non-invasive textile substrate classification with apparatus according to the invention.

The drawings are provided for purposes of illustration only and merely depict typical or example embodiments. These drawings are provided to facilitate the reader's understanding and shall not be considered limiting of the breadth, scope, or applicability of various embodiments.

The apparatus comprises the housing 2 which contains at least one emitter which emits the majority of its energy in NIR/SWIR range of electromagnetic spectrum from 900 nm to 2500 nm, and at least one light emitter which emits the majority of the energy in the visible spectrum (VIS) from 400 nm to 700nm. The apparatus also contains at least one detector 7 in NIR/SWIR wavelength range.

The optical module 5, which is positioned between the NIR/SWIR opening aperture 3 and NIR/SWIR detector 7 contains -according to the proposed invention- a compact diffused light collection and collimation optics with the two aspheric lenses 12 and 13 with different numerical apertures, and with the slit 14, carefully positioned between the two lenses at the position of the joint focal point F of both lenses. One or both of the aspheric lenses 12 and 13 can be replaced by the optical assembly of one or more spheric lenses thus resulting in the same optical beam condensing, narrowing, and collimating operation. The shape of the slit or aperture 14 is not limited and can be either circular, rectangular, square, elliptical, star-shaped, etc. The position (distance) of the two aspheric lenses, each placed on each side of the slit, is thus defined by the respective focal lengths of the two lenses.

The critical requirement according to this invention is the difference of numeric aperture (N.A.) values of the two lenses in such a way that the N.A. of the lens 12 facing the fabric classification apparatus input aperture 3 needs to be lower than the N.A. of the lens 13 facing the NIR/SWIR detector 7.

Furthermore, the apparatus also contains a proximity sensor 4, which is one of the crucial components of the apparatus according to this invention. The purpose of the proximity sensor is to detect the presence of the measured material 1 (textile substrate) near the predefined distance from the VIS and NIR/SWIR aperture 3. By using the proximity sensor, the spectroscopic analysis of the textile substrate is performed only when the textile substrate gets positioned at the ideal distance from the main spectrometer aperture. Namely, the quality of the reflected spectrometry heavily depends on the positioning of the textile substrate against the spectrometer aperture due to the following two reasons: the textile substrate needs to be first properly and sufficiently illuminated with the VIS/NIR/SWIR light source(s), and the diffusely reflected light needs to be picked up with the VIS/NIR/SWIR light detector. Any mispositioning will thus decrease the signal-to-noise ratio and consequently adversely affects the textile substrate classification efficiency. This invention does not limit the proximity sensor type. It can either be IR-LED based, laser-based, LiDAR-based, ultrasound-based, photo-electric, capacitive, inductive, radar-based, or Doppler-effect based. The usage of proximity sensors is otherwise well-known in the mobile phone industry: the proximity sensor in the mobile phone detects when the mobile phone is placed to the user's ear thus dimming the illumination of the main phone's display. The application of the proximity sensor in the textile substrate classification apparatus is, however, new and presents the inventive step of the proposed patent. Furthermore, the proximity sensor handling is seamlessly integrated with the proposed textile substrate classification procedure itself.

The central component of the proposed textile substrate classification apparatus is the central processor 9, which executes the fabric classification algorithm in the form of the computer program compiled and linked into the machine executable code, or is described with the hardware description language (HDL). The type of the central processor itself is thus not limited by this patent. It can either be a general purpose microprocessor, microcontroller, FPGA, System-on-Chip (SoC), or dedicated application specific integrated circuit (ASIC).

The procedure of textile substrate classification operates according to this invention along the following steps:
1. Start the algorithm 100.
2. Start NIR/SWIR illumination pre-heating 101 and enable proximity detector 102. The enabled proximity detector will trigger the measurement of the spectral reflectance response when the textile substrate gets positioned closer to the device's aperture 3. The purpose of NIR/SWIR illumination pre-heating is to shorten the overall time needed for the spectral measurement sequence once the proximity sensor detects the textile substrate presence. Namely, for accurate spectral measurement, the NIR/SWIR light emitter needs to be switched on for a certain time period before the spectral measurements start in order to assure its stable illumination flux. This time delay can be shortened if the light source is pre-heated with the fraction of the nominal operational current.
3. Once the proximity detector detects the presence of the textile substrate 103, first the NIR/SWIR illumination source gets full power 104. The spectral characteristic measurement can -according to this invention- operate in the two different configurations 105:
   a. The broadband NIR/SWIR emitter is utilized. In this case, the wavelength selection and narrow band filtering is done at NIR/SWIR detector's side (either by applying MOEMS Fabry-Perot interferometer placed in front of a single-point broadband detector, linear wavelength filter placed in front of a linear detector array, or by applying discrete wavelength filters for each of the multiple individual single point detectors positioned in the array or matrix configuration). The spectral response is collected either by varying the transmissive wavelength of the MOEMS Fabry-Perot interferometer and synchronously reading the output value from the single point detector or by reading the outputs of the multiple detectors configured either in a linear array or matrix configuration.
   b. Multiple narrowband NIR/SWIR emitters and a single broadband NIR/SWIR detector are utilized. In this case one or multiple narrowband emitters need to be sequentially switched on such that in a certain point in time only emitter(s) operating at the same central wavelength are active. The output value from the single broadband NIR/SWIR detector is then sequentially read in order to have the representation of the complete spectral response (the resolution is in this case defined by the number of narrowband emitters and the width (FWHM, Full-Width at Half-Maximum) of individual NIR/SWIR emitter).
4. Once the spectral measurement 105 is complete, the full lamp power is switched off 106, and the feature extraction procedure starts. The proposed feature extraction procedure is another vital inventive step of this invention and assures superior textile substrate recognition results. The feature extraction operates as follows:
   a. As a first step of the feature extraction procedure, a spectral response smoothing by a moving average (MA) filter is performed 107. The purpose of this step is to reduce any sample-to-sample variations due to any time variations occurring during sampling of the spectrum. The MA filtering thus helps to improve the signal-to-noise ratio (SNR).
   b. Next, the root-mean-square (RMS) energy normalization 108 of the smoothed spectral response is performed. The purpose of this step is to normalize all captured spectral responses to the same overall energy level. This step is crucial in order to eliminate the signal energy dependency from the classification performance. The textile substrate classification will thus not be affected if -for example- the NIR/SWIR illumination flux changes due to lamps wearing out.
   c. The final step of NIR/SWIR spectral feature extraction is decorrelation operation, which reduces the autocorrelation within the feature vector. The decorrelation of the NIR/SWIR spectral feature vector elements is according to this invention performed by means of Discrete Cosine Transform (DCT) 109. Furthermore, the decorrelation by DCT also makes the covariance matrix of NIR/SWIR spectral feature vector elements diagonal, and sorts the feature vector elements in the descending order with respect to covariance values along the diagonal. Thus it is possible to reduce the dimension of the output feature vector by retaining only the feature vector elements with largest covariances and discard the elements which have lower covariance values thus containing less important information about the textile substrate composition. By this the complexity of the machine training and classification can be reduced by minimally affecting the classification performance.
   The above described NIR/SWIR spectral feature extraction and processing by means of DCT is another inventive step of this patent.
5. The extracted final output feature vector afterwards enters the machine classification algorithm 110 running on the central processor. The machine classification algorithm itself is also not limited by this patent. It can either be artificial neural network (ANN), deep neural network (DNN), gaussian mixture model (GMM), support vector machine (SVM), etc. In one typical application example, the machine learning algorithm can be trained by a supervised algorithm with the following 8 basic fabric substrate categories: cotton, wool, silk, viscose, polyester, polyamide, polyacrylonitrile, and polyurethane. The combination (mixing) of two or more fabric substrates is also possible (e.g. 65 % cotton + 35 % polyester). An additional, ninth output class can be specified as a garbage model, which represents and models all other materials which could be placed accidentally in front of the sensor aperture (e.g. some metal items, human hand, etc.). An alternative to the garbage model is also a confidence measure. The combination of both the garbage model and the confidence measure is also possible in order to further increase the classification robustness.
6. The final textile substrate classification score is communicated 111 toward the host apparatus via contact or contactless interface.
7. If repetitive mode is enabled, another proximity detection can be triggered, thus sampling another textile substrate automatically 112. Alternatively, the algorithm can terminate 113, thus waiting for another retrigger of the algorithm from the host apparatus.

The proposed textile substrate classification apparatus also contains a communication interface 10, which is internally connected to the central processing unit 9. The communication mode itself is not limited by this invention. It can be either contact-based (UART, RS232, RS485, USB, CAN, LVDS, etc.) or contactless (radiofrequency (WiFi, Bluetooth, Bluetooth Low Energy, ZigBee, GPRS, LTE), or optical (IrDA)). An important part of the apparatus is also the power supply unit 11. In the case of contactless communication, the apparatus contains an autonomous power supply through (re)chargeable or replaceable batteries. In case of contact communication, the power is supplied externally. In the particular case of USB contact communication, the power is supplied through this standard interface without the need to attach an additional, separate power supply cable.

## Claims

1. Proximity triggered textile substrate classification apparatus,
**characterized by** a housing (2) with NIR/SWIR transparent window (3), underneath containing a proximity sensor (4), at least one broadband NIR/SWIR illumination emitter (8), optical unit (5), at least one broadband NIR/SWIR detector (6, 7), central control and processing unit (9), power supply unit (11), and communication interface (10)wherein,
the optical unit (5) is positioned between the NIR/SWIR transparent window (3) and the at least one broadband NIR/SWIR detector (6, 7) and comprises two aspheric lenses (12,13), and an aperture (14), wherein:
a. the first aspheric lens (12) is facing the transparent window (3) and has a numeric aperture (N.A.) lower than the numeric aperture of the second aspheric lens (13) facing the NIR/SWIR detector (6, 7);
b. the aperture (14) is positioned between the first and the second aspheric lens such that the central distance of the aperture from both aspheric lenses matches the focal lengths of the two aspheric lenses respectively;
c. the first aspheric lens (12), the aperture (14), and the second aspheric lens (13) are aligned along the same central axis,
the proximity sensor (4) **is** arranged to autonomously trigger the reflectance spectral response measurement and textile substrate classification only when the textile substrate reaches the ideal position against the NIR/SWIR transparent window (3).

2. Proximity triggered textile substrate classification apparatus according to claim 1,
**characterized by** the proximity sensor (4) which can be IR-LED based, laser-based, LiDAR-based, ultrasound-based, photo-electric, capacitive, inductive, radar-based, or Doppler-effect based.

3. Proximity triggered textile substrate classification apparatus according to claim 1 or 2,
**characterized by** the alternative implementation with multiple narrowband NIR/SWIR emitters, such that sequentially only the NIR/SWIR emitters operating in the same wavelength band are switched-on at a time, and with one broadband NIR/SWIR detector.

4. Proximity triggered textile substrate classification apparatus according to claims 1 to 3,
**characterized by** the central processing unit which implements the textile substrate machine classification algorithm in the form of hardware description language (HDL) and operates inside a Field Programmable Gate Array (FPGA) integrated circuit.

5. Proximity triggered textile substrate classification apparatus according to claims 1 to 4,
**characterized in that** the central control and processing unit (9) is arranged to carry out the NIR/SWIR feature extraction procedure for textile substrate classification consisting of the following steps:
a. the reflective NIR/SWIR spectral response is smoothed with moving average (MA) low-pass filter;
b. root mean square (RMS) energy normalization of the smoothed spectral response is performed;
c. cepstrum of the RMS normalized smoothed spectral response is calculated by means of discrete cosine transform;
d. a resulting vector of cepstral features is used in textile substrate machine classification algorithm.

6. Proximity triggered textile substrate classification apparatus according to claims 1 to 5,
**characterized by** utilizing less than 20 wavelength bands in the region 900 nm to 2500 nm as an input to discrete cosine transform (DCT).

7. Proximity triggered textile substrate classification apparatus according to claims 1 to 6,
**characterized by** the central processing unit which implements the textile substrate machine classification algorithm in the form of ASIC.

8. Proximity triggered textile substrate classification apparatus according to claims 1 to 7, further comprising a colour (VIS) sensor.

9. Proximity triggered textile substrate classification apparatus according to claim 1,
wherein the shape of the aperture (14) is either circular, rectangular, square, elliptical, or star-shaped.

10. Proximity triggered textile substrate classification apparatus according to claim 1,
**characterized by** a possible implementation of the optical module (5), where one or both of the aspheric lenses (12,13) can be replaced by the optical assembly of one or more spherical lenses (12,13) thus resulting in the same optical beam condensing, narrowing, and collimating operation.

11. Procedure for non-invasive textile substrate classification with apparatus according to claims 1 to 10,
**characterized by**
a. proximity sensor triggered NIR/SWIR reflectance spectrum measurement by switching-on at least one NIR/SWIR emitter (8) and measuring the reflectance spectrum response from at least one NIR/SWIR detector (6),
b. generating the feature vector by postprocessing the NIR/SWIR reflectance spectrum response by means of moving average filtering, root-mean-square energy normalization, and discrete cosine transformation,
c. classifying the textile substrate using the generated NIR/SWIR feature vector in multiple classification classes out of which one class represents the garbage collection class in combination with a confidence value,
d. transmitting the textile substrate classification response to the host device by either contact or contactless connection.

## Patentansprüche

1. Näherungsgesteuerte Vorrichtung zur Klassifizierung von Textilsubstraten,
**gekennzeichnet durch**
ein Gehäuse (2) mit einem NIR/SWIR-transparenten Fenster (3), unterhalb dessen ein Näherungssensor (4), mindestens ein Breitband-NIR/SWIR-Emitter (8), eine optische Einheit (5), mindestens ein Breitband-NIR/SWIR-Detektor (6, 7), eine zentrale Steuer- und Verarbeitungseinheit (9), eine Stromversorgungseinheit (11) und eine Kommunikationsschnittstelle (10) angeordnet sind, wobei die optische Einheit (5) zwischen dem NIR/SWIR-transparenten Fenster (3) und dem mindestens einen Breitband-NIR/SWIR-Detektor (6, 7) angeordnet ist und zwei asphärische Linsen (12, 13) sowie eine Apertur (14) umfasst, wobei:
a. die erste asphärische Linse (12) dem transparenten Fenster (3) zugewandt ist und eine numerische Apertur (N.A.) aufweist, die geringer ist als die numerische Apertur der zweiten asphärischen Linse (13), die dem NIR/SWIR-Detektor (6, 7) zugewandt ist;
b. die Apertur (14) zwischen der ersten und der zweiten asphärischen Linse so angeordnet ist, dass der Abstand der Apertur zu beiden asphärischen Linsen jeweils den Brennweiten der beiden asphärischen Linsen entspricht;
c. die erste asphärische Linse (12), die Apertur (14) und die zweite asphärische Linse (13) entlang derselben Mittelachse ausgerichtet sind,
der Näherungssensor (4) so angeordnet ist, dass er die Messung der spektralen Reflexionsantwort und die Klassifizierung des Textilsubstrats nur dann autonom auslöst, wenn das Textilsubstrat die ideale Position gegenüber dem transparenten NIR/SWIR-Fenster (3) erreicht.

2. Näherungsgesteuerte Vorrichtung zur Klassifizierung von Textilsubstraten nach Anspruch 1,
**gekennzeichnet durch**
den Näherungssensor (4), der als IR-LED-, Laser-, LiDAR-, Ultraschall-, photoelektrischer, kapazitiver, induktiver, radarbasierter oder auf dem Doppler-Effekt beruhender Sensor ausgebildet sein kann.

3. Näherungsgesteuerte Vorrichtung zur Klassifizierung von Textilsubstraten nach Anspruch 1 oder 2,
**gekennzeichnet durch**
die alternative Implementierung mit mehreren Schmalband-NIR/SWIR-Emittern derart, dass sequentiell jeweils nur die in demselben Wellenlängenband arbeitenden NIR/SWIR-Emitter eingeschaltet werden, und mit einem Breitband-NIR/SWIR-Detektor.

4. Näherungsgesteuerte Vorrichtung zur Klassifizierung von Textilsubstraten nach Ansprüchen 1 bis 3,
**gekennzeichnet durch**
die zentrale Verarbeitungseinheit, die den Algorithmus zur maschinellen Klassifizierung von Textilsubstraten in der Form einer Hardware Description Language (HDL) implementiert und innerhalb einer integrierten Schaltung mit einem Field Programmable Gate Array (FPGA) arbeitet.

5. Näherungsgesteuerte Vorrichtung zur Klassifizierung von Textilsubstraten nach Ansprüchen 1 bis 4,
**gekennzeichnet durch**
die Einrichtung der zentralen Steuer- und Verarbeitungseinheit (9) zur Durchführung des NIR/SWIR-Merkmalsextraktionsverfahrens für die Klassifizierung von Textilsubstraten, das aus den folgenden Schritten besteht:
a. Glättung der spektralen Reflexionsantwort in dem NIR/SWIR-Bereich mit einem Moving Average (MA)-Tiefpassfilter;
b. Durchführung der Root Mean Square (RMS)-Energienormalisierung der geglätteten spektralen Reflexionsantwort;
c. Berechnung des Cepstrums der geglätteten, RMS-normalisierten spektralen Reflexionsantwort mittels diskreter Kosinustransformation;
d. Verwendung eines resultierenden Vektors cepstraler Merkmale in dem Algorithmus zur maschinellen Klassifizierung von Textilsubstraten.

6. Näherungsgesteuerte Vorrichtung zur Klassifizierung von Textilsubstraten nach Ansprüchen 1 bis 5,
**gekennzeichnet durch**
die Verwendung von weniger als 20 Wellenlängenbändern in dem Bereich von 900 nm bis 2500 nm als eine Eingabe für die Discrete Cosine Transformation (DCT).

7. Näherungsgesteuerte Vorrichtung zur Klassifizierung von Textilsubstraten nach Ansprüchen 1 bis 6,
**gekennzeichnet durch**
die zentrale Verarbeitungseinheit, die den Algorithmus zur maschinellen Klassifizierung von Textilsubstraten in der Form eines ASIC implementiert.

8. Näherungsgesteuerte Vorrichtung zur Klassifizierung von Textilsubstraten nach Ansprüchen 1 bis 7, ferner umfassend
einen Farbsensor (VIS).

9. Näherungsgesteuerte Vorrichtung zur Klassifizierung von Textilsubstraten nach Anspruch 1,
wobei die Form der Apertur (14) entweder kreisförmig, rechteckig, quadratisch, elliptisch oder sternförmig ist.

10. Näherungsgesteuerte Vorrichtung zur Klassifizierung von Textilsubstraten nach Anspruch 1,
**gekennzeichnet durch**
eine mögliche Implementierung des optischen Moduls (5), bei der eine oder beide der asphärischen Linsen (12, 13) durch die optische Anordnung einer oder mehrerer sphärischer Linsen (12, 13) ersetzt werden können, wodurch dieselbe optische Strahlbündelung, Strahlverengung und Strahlkollimation erzielt wird.

11. Verfahren zur nicht-invasiven Klassifizierung von Textilsubstraten mit einer Vorrichtung nach Ansprüchen 1 bis 10,
**gekennzeichnet durch**
a. eine durch einen Näherungssensor ausgelöste Messung des NIR/SWIR-Reflexionsspektrums durch Einschalten mindestens eines NIR/SWIR-Emitters (8) und Erfassung der spektralen Reflexionsantwort von mindestens einem NIR/SWIR-Detektor (6),
b. Erzeugung des Merkmalsvektors durch Nachbearbeiten der spektralen NIR/SWIR-Reflexionsantwort mittels Moving Average Filterung, Root Mean Square Energienormalisierung und Discrete Cosine Transformation,
c. Klassifizierung des Textilsubstrats unter Verwendung des erzeugten NIR/SWIR-Merkmalsvektors in mehrere Klassifizierungsklassen, von denen eine Klasse die Abfallklasse in Kombination mit einem Konfidenzwert darstellt,
d. Übertragung des Textilsubstrat-Klassifizierungsergebnisses an das Hostgerät durch entweder kontaktgebundene oder kontaktlose Verbindung.

## Revendications

1. Appareil de classification de substrats textiles déclenché par proximité,
**caractérisé par** un boîtier (2) avec une fenêtre transparente NIR/SWIR (3), contenant en dessous un capteur de proximité (4), au moins un émetteur d'éclairage NIR/SWIR (8) à large bande, une unité optique (5), au moins un détecteur NIR/SWIR (6, 7) à large bande, une unité centrale (9) de commande et de traitement, une unité (11) d'alimentation électrique et une interface de communication (10),
l'unité optique (5) étant positionnée entre la fenêtre transparente NIR/SWIR (3) et lesdits un ou plusieurs détecteurs NIR/SWIR (6, 7) à large bande et comprenant deux lentilles asphériques (12, 13) et une ouverture (14), dans lequel :
a. la première lentille asphérique (12) est tournée vers la fenêtre transparente (3) et a une ouverture numérique (N.A.) inférieure à l'ouverture numérique de la deuxième lentille asphérique (13) tournée vers le détecteur NIR/SWIR (6, 7) ;
b. l'ouverture (14) est positionnée entre la première et la deuxième lentille asphérique de telle sorte que la distance centrale de l'ouverture par rapport aux deux lentilles asphériques corresponde respectivement aux distances focales des deux lentilles asphériques ;
c. la première lentille asphérique (12), l'ouverture (14) et la deuxième lentille asphérique (13) sont alignées le long du même axe central,
le capteur de proximité (4) est agencé de façon à déclencher de manière autonome la mesure de la réponse spectrale de réflectance et la classification du substrat textile uniquement lorsque le substrat textile atteint la position idéale par rapport à la fenêtre transparente NIR/SWIR (3).

2. Appareil de classification de substrats textiles déclenché par proximité selon la revendication 1, **caractérisé par le fait que** le capteur de proximité (4) peut être à base de DEL IR, à base de laser, à base de LIDAR, à base d'ultrasons, photoélectrique, capacitif, inductif, à base de radar ou à base d'effet Doppler.

3. Appareil de classification de substrats textiles déclenché par proximité selon la revendication 1 ou la revendication 2, **caractérisé par** la mise en œuvre alternative avec plusieurs émetteurs NIR/SWIR à bande étroite, de telle sorte que seuls les émetteurs NIR/SWIR fonctionnant dans la même bande de longueurs d'onde soient activés séquentiellement à la fois, et avec un détecteur NIR/SWIR à large bande.

4. Appareil de classification de substrats textiles déclenché par proximité selon les revendications 1 à 3, **caractérisé par le fait que** l'unité centrale de traitement met en œuvre l'algorithme de classification des machines à substrats textiles sous la forme d'un langage de description du matériel (HDL) et fonctionne à l'intérieur d'un circuit intégré de type FPGA (Field-Programmable Gate Array).

5. Appareil de classification de substrats textiles déclenché par proximité selon les revendications 1 à 4, **caractérisé en ce que** l'unité centrale (9) de commande et de traitement est agencée de manière à effectuer une procédure d'extraction de caractéristiques NIR/SWIR pour la classification de substrats textiles, comprenant les étapes suivantes :
a. la réponse spectrale réfléchissante NIR/SWIR est lissée au moyen d'un filtre passe-bas à moyenne mobile (MA) ;
b. une normalisation de l'énergie quadratique moyenne (RMS) de la réponse spectrale lissée est effectuée ;
c. le cepstre de la réponse spectrale RMS lissée normalisée est calculé au moyen d'une transformée cosinus discrète ;
d. un vecteur résultant des caractéristiques cepstrales est utilisé dans un algorithme de classification des substrats textiles.

6. Appareil de classification de substrats textiles déclenché par proximité selon les revendications 1 à 5,
**caractérisé par** l'utilisation de moins de 20 bandes de longueurs d'onde dans la zone de 900 nm à 2500 nm comme entrée pour la transformée cosinus discrète (DCT).

7. Appareil de classification de substrats textiles déclenché par proximité selon les revendications 1 à 6,
**caractérisé par le fait que** l'unité centrale de traitement met en oeuvre l'algorithme de classification des machines à substrats textiles sous la forme d'un ASIC.

8. Appareil de classification de substrats textiles déclenché par proximité selon les revendications 1 à 7, comprenant en outre un capteur de couleur (VIS).

9. Appareil de classification de substrats textiles déclenché par proximité selon la revendication 1, dans lequel la forme de l'ouverture (14) est circulaire, rectangulaire, carrée, elliptique ou en forme d'étoile.

10. Appareil de classification de substrats textiles déclenché par proximité selon la revendication 1, **caractérisé par** une mise en oeuvre possible du module optique (5), dans lequel l'une ou les deux lentilles asphériques (12, 13) peuvent être remplacées par l'ensemble optique d'une ou plusieurs lentilles sphériques (12, 13), ce qui permet d'obtenir le même fonctionnement de condensation, de rétrécissement et de collimation du faisceau optique.

11. Procédé de classification non invasive de substrats textiles au moyen d'un appareil selon les revendications 1 à 10,
**caractérisé par**
a. la mesure du spectre de réflectance NIR/SWIR déclenchée par un capteur de proximité en activant au moins un émetteur NIR/SWIR (8) et en mesurant la réponse du spectre de réflectance à partir d'au moins un détecteur NIR/SWIR (6),
b. la génération du vecteur caractéristique par post-traitement de la réponse du spectre de réflectance NIR/SWIR au moyen d'un filtrage par moyenne mobile, d'une normalisation d'énergie quadratique moyenne, et d'une transformation cosinus discrète,
c. la classification du substrat textile au moyen du vecteur de caractéristiques NIR/SWIR généré dans plusieurs classes de classification, dont une classe représente la classe de collecte des déchets en combinaison avec une valeur de confiance,
d. la transmission de la réponse de classification du substrat textile au dispositif hôte par connexion avec ou sans contact.
